# EUROPEAN PATENT SPECIFICATION

(11) **EP 1 385 551 B1**
(45) Date of publication and mention of the grant of the patent: **03.09.2008**
(21) Application number: 02726710.3
(22) Date of filing: 05.04.2002
(51) Int. Cl.: A61K 31/436, A61K 31/7068, A61K 31/505, A61K 31/513, A61K 31/519, A61P 35/00

(54) **ANTINEOPLASTIC COMBINATIONS COMPRISING CCI-779 (RAPAMYCIN DERIVATIVE) TOGETHER WITH GEMCITABINE OR FLUOROURACIL**
ANTINEOPLASTISCHE KOMBINATIONSPRÄPARATE ENTHALTEND CCI-779 (RAPAMYCIN DERIVAT) ZUSAMMEN MIT GEMCITABIN ODER FLUORO-URACIL
COMBINAISONS ANTINEOPLASIQUES CONTENANTS DU CCI-779 (DERIVÉE DE RAPAMYCINE) ASSOCIE A LA GEMCITABINE OU AU FLUOROURACILE

(30) Priority: 06.04.2001 US 282385 P; 06.04.2001 US 282388 P
(43) Date of publication of application: 04.02.2004
(73) Proprietor: Wyeth, Madison, NJ 07940-0874 (US)
(72) Inventor: GIBBONS, James, Joseph, Jr., Westwood, NJ 07675 (US); DUKART, Gary, Ambler, PA 19002 (US); FRISCH, Jurgen, Hermann, Ernst, 35041 Marburg (DE)
(74) Representative: Wileman, David Francis
(86) International application number: PCT/US2002/010912
(87) International publication number: WO 2002/080975

(56) References cited:
- WO-A-99/52514
- US-A- 5 066 493
- ENG C P ET AL: "Activity of rapamycin (AY-22,989) against transplanted tumors." THE JOURNAL OF ANTIBIOTICS. JAPAN OCT 1984, vol. 37, no. 10, October 1984 (1984-10), pages 1231-1237, XP001098253 ISSN: 0021-8820
- ALEXANDRE J ET AL: "LA RAPAMYCINE ET LE CCI-779//RAPAMYCIN AND CCI-779" CANCER BULLETIN, MEDICAL ARTS PUB, HOUSTON, US, vol. 10, no. 86, October 1999 (1999-10), pages 808-811, XP001078856 ISSN: 0008-5448
- ALEXANDRE J ET AL: "PHASE I STUDY OF CCI-779,A NOVEL RAPAMYCIN ANALOG: PRELIMINARY RESULTS" PROCEEDINGS OF THE ANNUAL MEETING OF THE AMERICAN ASSOCIATION FOR CANCER RESEARCH, NEW YORK, NY, US, vol. 41, no. 41, March 2000 (2000-03), page 613 XP001071217 ISSN: 0197-016X
- PUNT C J A ET AL: "A phase I study of escalating doses of CCI-779 in combination with 5-fluorouracil and leucovorin in patients with advanced solid tumors." EUROPEAN JOURNAL OF CANCER, vol. 37, no. Supplement 6, October 2001 (2001-10), page S17 XP001093978 11th European Cancer Conference;Lisbon, Portugal; October 21-25, 2001, October, 2001 ISSN: 0959-8049
- BONI J ET AL: "Pharmacokinetics of escalating doses of CCI-779 in combination with 5-fluorouracil and leucovorin in patients with advanced solid tumors." EUROPEAN JOURNAL OF CANCER, vol. 37, no. Supplement 6, October 2001 (2001-10), page S68 XP001093977 11th European Cancer Conference;Lisbon, Portugal; October 21-25, 2001, October, 2001 ISSN: 0959-8049

## Description

This invention relates to antineoplastic combinations, more particularly to the use of combinations of an mTOR inhibitor (rapamycin 42-ester with 3-hydroxy-2-(hydroxymethyl)-2-methylpropionic acid (CCI-779)) and an antimetabolite antineoplastic agent selected from 5-fluorouracil and gemcitabine in the treatment of neoplasms.

### BACKGROUND OF THE INVENTION

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Sehgal et al., J. Antibiot. 28, 727 (1975); H. A. Baker et al., J. Antibiot. 31. 539 (1978); U.S. Patent 3,929,992; and U.S. Patent 3,993,749]. Additionally, rapamycin alone (U.S. Patent 4,885,171) or in combination with picibanil (U.S. Patent 4,401,653) has been shown to have antitumor activity.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); R. Y. Calne et al., Lancet 1183 (1978); and U.S. Patent 5,100,899]. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

Rapamycin is also useful in preventing or treating systemic lupus erythematosus [U.S. Patent 5,078,999], pulmonary inflammation [U.S. Patent 5,080,899], insulin dependent diabetes mellitus [U.S. Patent 5,321,009], skin disorders, such as psoriasis [U.S. Patent 5,286,730], bowel disorders [U.S. Patent 5,286,731], smooth muscle cell proliferation and intimal thickening following vascular injury [U.S. Patents 5,288,711 and 5,516,781], adult T-cell leukemia/lymphoma [European Patent Application 525,960 A1], ocular inflammation [U.S. Patent 5,387,589], malignant carcinomas [U.S. Patent 5,206,018], cardiac inflammatory disease [U.S. Patent 5,496,832], and anemia [U.S. Patent 5,561,138].

Rapamycin 42-ester with 3-hydroxy-2-(hydroxymethyl)-2-methylpropionic acid (CCI-779) is ester of rapamycin which has demonstrated significant inhibitory effects on tumor growth in both in vitro and in vivo models. The preparation and use of hydroxyesters of rapamycin, including CCI-779, are disclosed in U.S. Patent 5,362,718.

CCI-779 exhibits cytostatic, as opposed to cytotoxic properties, and may delay the time to progression of tumors or time to tumor recurrence. CCI-779 is considered to have a mechanism of action that is similar to that of sirolimus. CCI-779 binds to and forms a complex with the cytoplasmic protein FKBP, which inhibits an enzyme, mTOR (mammalian target of rapamycin, also known as FKBP12-rapamycin associated protein [FRAP]). Inhibition of mTOR's kinase activity inhibits a variety of signal transduction pathways, including cytokine-stimulated cell proliferation, translation of mRNAs for several key proteins that regulate the G1 phase of the cell cycle, and IL-2-induced transcription, leading to inhibition of progression of the cell cycle from G1 to S. The mechanism of action of CCI-779 that results in the G1→ S phase block is novel for an anticancer drug.

In vitro, CCI-779 has been shown to inhibit the growth of a number of histologically diverse tumor cells. Central nervous system (CNS) cancer, leukemia (T-cell), breast cancer, prostate cancer, and melanoma lines were among the most sensitive to CCI-779. The compound arrested cells in the G1 phase of the cell cycle.

In vivo studies in nude mice have demonstrated that CCI-779 has activity against human tumor xenografts of diverse histological types. Gliomas were particularly sensitive to CCI-779 and the compound was active in an orthotopic glioma model in nude mice. Growth factor (platelet-derived)-induced stimulation of a human glioblastoma cell line in vitro was markedly suppressed by CCI-779. The growth of several human pancreatic tumors in nude mice as well as one of two breast cancer lines studied in vivo also was inhibited by CCI-779.

### DESCRIPTION OF THE INVENTION

This invention provides the use of combinations of an mTOR inhibitor and an antimetabolite antineoplastic agent as antineoplastic combination chemotherapy. In particular, these combinations are useful in the treatment of renal cancer, soft tissue cancer, breast cancer, neuroendocrine tumor of the lung, cervical cancer, uterine cancer, head and neck cancer, glioma, non-small lung cell cancer, prostate cancer, pancreatic cancer, lymphoma, melanoma, small cell lung cancer, ovarian cancer, colon cancer, esophageal cancer, gastric cancer, leukemia, colorectal cancer, and unknown primary cancer. This invention also provides combinations of an mTOR inhibitor and an antimetabolite antineoplastic agent for use as antineoplastic combination chemotherapy, in which the dosage of either the mTOR inhibitor or the antimetabolite antineoplastic agent or both are used in subtherapeutically effective dosages.

As used in accordance with this invention, the term "treatment" means treating a mammal having a neoplastic disease by providing said mammal an effective amount of a combination of an mTOR inhibitor and an antimetabolite antineoplastic agent with the purpose of inhibiting growth of the neoplasm in such mammal, eradication of the neoplasm, or palliation of the mammal.

As used in accordance with this invention, the term "providing," with respect to providing the combination, means either directly administering the combination, or administering a prodrug, derivative, or analog of one or both of the components of the combination which will form an effective amount of the combination within the body.

mTOR is the mammalian target of rapamycin, also known as FKBP12-rapamycin associated protein [FRAP]. Inhibition of mTOR's kinase activity inhibits a variety of signal transduction pathways, including cytokine-stimulated cell proliferation, translation of mRNAs for several key proteins that regulate the G1 phase of the cell cycle, and IL-2-induced transcription, leading to inhibition of progression of the cell cycle from G1 to S.

mTOR regulates the activity of at least two proteins involved in the translation of specific cell cycle regulatory proteins (Bumett, P.E., PNAS 95: 1432 (1998) and Isotani, S., J. Biol. Chem. 274: 33493 (1999)). One of these proteins p70s6 kinase is phosphorylated by mTOR on serine 389 as well as threonine 412. This phosphorylation can be observed in growth factor treated cells by Western blotting of whole cell extracts of these cells with antibody specific for the phosphoserine 389 residue.

As used in accordance with this invention, an "mTOR inhibitor" means a compound or ligand which inhibits cell replication by blocking progression of the cell cycle from G1 to S by inhibiting the phosphorylation of serine 389 of p70s6 kinase by mTOR.

The following standard pharmacological test procedure can be used to determine whether a compound is an mTOR inhibitor, as defined herein. Treatment of growth factor stimulated cells with an mTOR inhibitor like rapamycin completely blocks phosphorylation of serine 389 as evidenced by Western blot and as such constitutes a good assay for mTOR inhibition. Thus whole cell lysates from cells stimulated by a growth factor (eg. IGF1) in culture in the presence of an mTOR inhibitor should fail to show a band on an acrylamide gel capable of being labeled with an antibody specific for serine 389 of p70s6K.

### Materials:

| | |
|---|---|
| NuPAGE LDS Sample Buffer | (Novex Cat # NP0007) |
| NuPAGE Sample Reducing Agent | (Novex Cat # NP0004) |
| NuPAGE 4-12% Bis-Tris Gel | (Novex Cat # NP0321) |
| NuPAGE MOPS SDS Running Buffer | (Novex Cat # NP0001) |
| Nitrocellulose | (Novex Cat # LC2001) |
| NuPAGE Transfer Buffer | (Novex Cat # NP0006) |
| Hyperfilm ECL | (Amersham Cat # RPN3114H) |
| ECL Western Blotting Detection Reagent | (Amersham Cat # RPN2134) |
| | |
| Primary antibody: Phospho-p70 S6 Kinase (Thr389) | (Cell Signaling Cat # 9205) |
| Secondary antibody: Goat anti-rabbit IgG-HRP conjugate | (Santa Cruz Cat # sc-2004) |

### Methods:

### A. Preparation of Cell Lysates

Cell lines were grown in optimal basal medium supplemented with 10% fetal bovine serum and penicillin/treptomycin. For phosphorylation studies, cells were subcultured in 6-well plates. After the cells have completely attached, they were either serum-starved. Treatment with mTOR inhibitors ranged from 2 to 16 hours. After drug treatment, the cells were rinsed once with PBS (phosphate buffered saline without Mg++ and Ca++) and then lysed in 150-200 µl NuPAGE LDS sample buffer per well. The lysates were briefly sonicated and then centrifuged for 15 minutes at 14000 rpm. Lysates were stored at minus -80°C until use.

The test procedure can also be run by incubating the cells in growth medium overnigh, after they have completely attached. The results under both sets of conditions should be the same for an mTOR inhibitor.

### B. Western Blot Analysis

1) Prepare total protein samples by placing 22.5 µl of lysate per tube and then add 2.5 µl NuPAGE sample reducing agent. Heat samples at 70°C for 10 minutes. Electrophoresed using NuPAGE gels and NuPAGE SDS buffers.
2) Transfer the gel to a nitrocellulose membrane with NuPAGE transfer buffer. The membrane are blocked for 1 hour with blocking buffer (Tris buffered saline with 0.1 %-Tween and 5% nonfat-milk). Rinse membranes 2x with washing buffer (Tris buffered saline with 0. 1 %-Tween).
3) Blots/membrane are incubated with the P-p70 S6K (T389) primary antibody (1:1000) in blocking buffer overnight at 4°C in a rotating platform.
4) Blots are rinsed 3x for 10 minutes each with washing buffer, and incubated with secondary antibody (1:2000) in blocking buffer for 1 hour at room temperature.
5) After the secondary antibody binding, blots are washed 3x for 10 minutes each with washing buffer, and 2x for 1 minute each with Tris-buffered saline, followed by chemiluminescent (ECL) detection and then exposed to chemiluminescence films.

As described herein, CCI-779 is used as a mTOR inhibitor in the mTOR inhibitor plus antimetabolite combinations of this invention.

The preparation of CCI-779 is described in U.S. Patent 5,362,718. When CCI-779 is used as an antineoplastic agent, it is projected that initial i.v. infusion dosages will be between 0.1 and 100 mg/m² when administered on a daily dosage regimen (daily for 5 days, every 2-3 weeks), and between 0.1 and 1000 mg/m² when administered on a once weekly dosage regimen. Oral or intravenous infusion are the preferred routes of administration, with intravenous being more preferred.

As used in accordance with this invention, the term "antimetabolite" means a substance which is structurally similar to a critical natural intermediate (metabolite) in a biochemical pathway leading to DNA or RNA synthesis which is used by the host in that pathway, but acts to inhibit the completion of that pathway (i.e., synthesis of DNA or RNA). More specifically, antimetabolites typically function by (1) competing with metabolites for the catalytic or regulatory site of a key enzyme in DNA or RNA synthesis, or (2) substitute for a metabolite that is normally incorporated into DNA or RNA, and thereby producing a DNA or RNA that cannot support replication. Major categories of antimetabolites include (1) folic acid analogs, which are inhibitors of dihydrofolate reductase (DHFR); (2) purine analogs, which mimic the natural purines (adenine or guanine) but are structurally different so they competitively or irreversibly inhibit nuclear processing of DNA or RNA; and (3) pyrimidine analogs which mimic the natural pyrimidines (cytosine, thymidine, and uracil) but are structurally different so they competitively or irreversibly inhibit nuclear processing of DNA or RNA.

The following are useful as antimetabolites in this invention.

5-Fluorouracil (5-FU; 5-fluoro-2,4(1*H*,3*H*)-pyrimidinedione) is commercially available in a topical cream (FLUOROPLEX or EFUDEX) a topical solution (FLUOROPLEX or EFUDEX), and as an injectable containing 50 mg/mL 5-fluorouracil (ADRUCIL or flurouracil).

Gemcitabine (2'-deoxy-2',2'-difluorocytidine monohydrochloride ((beta)-isomer)), is commercially available as a liquid injectable containing between 200 mg - 1g/vial (GEMZAR).

The following table briefly summarizes some of the recommended dosages for the antimetabolites listed above.

| **Drug** | **Dosage** | **Regimen** |
|---|---|---|
| 5-Fluorouracil | 12 mg/kg oral | daily for 4 days |
| | 6 mg/kg oral | days 6, 8, 10, 12 |
| | | no drug on days 5, 7, 9, and 11; doses cut in half if toxicity observed |
| | 370 - 600 mg/m² i.v. | daily for 5 days, every 3-4 weeks |

| Floxuradine (FUDR) | 0.1-0.6 mg/kg | daily by arterial infusion |
|---|---|---|
| Gemcitabine (GEMZAR) | 1000 mg/m²/30 min | single agent once weekly for 7 weeks, followed by 1 week rest, then once weekly for 3 out of every 4 weeks |
| | 1000 -1250 mg/m²/ 30 min | combination therapy: days 1, 8, 15 per 28 day cycle, or days 1 and 8 per 21 day cycle |

This invention also covers the use of the mTOR inhibitor plus the antimetabolite in which a biochemical modifying agent is part of the chemotherapeutic regimen. The term "biochemical modifying agent" is well known and understood to those skilled in the art as an agent given as an adjunct to antimetabolite therapy, which serves to potentiate its antineoplastic activity, as well as counteract the side effects of the antimetabolite. Leucovorin and levofolinate are typically used as biochemical modifying agents for methotrexate and 5-FU therapy.

Leucovorin (5-formyl-5,6,7,8-tetrahydrofolic acid) is commercially available as an injectable liquid containing between 5 - 10 mg/mL or 50 - 350 mg/vial (leucovorin calcium or WELLCOVORIN) and as 5 - 25 mg oral tablets (leucovorin calcium).

Levofolinate (pharmacologically active isomer of 5-formyltetrahydrofolic acid) is commercially available as an injectable containing 25 - 75 mg levofolinate (ISOVORIN) or as 2.5 - 7.5 mg oral tablets (ISOVORIN).

Preferred mTOR inhibitor plus antimetabolite combinations of this invention include CCI-779 plus gemcitabine; CCI-779 plus 5-fluorouracil; and CCI-779 plus 5-fluorouracil plus leucovorin. It is preferred that the CCI-779 plus gemcitabine combination be used in treating pancreatic cancer and that the CCI-779 plus 5-fluorouracil combination (with or without leucovorin) be used in treating colorectal cancer.

The antineoplastic activity of the CCI-779 plus antimetabolite combination was confirmed in *in vitro* and *in vivo* standard pharmacological test procedures using combinations of CCI-779 plus gemcitabine; and CCI-779 plus 5-fluorouracil as representative combinations of this invention. The following briefly describes the procedures used and the results obtained.

Human rhabdomyosarcoma lines Rh30 and Rh1 and the human glioblastoma line SJ-GBM2 were used for *in vitro* combination studies with CCI-779 and antimetabolite agents. *In vivo* studies used a human neuroblastoma (NB1643) and human colon line GC3.

Dose response curves were determined for each of the drugs of interest. The cell lines Rh30, Rh1 and SJ-G2 were plated in six-well cluster plates at 6x10³, 5x10³ and 2.5x10⁴ cells/well respectively. After a 24 hour incubation period, drugs were added in either 10%FBS+RPMI 1640 for Rh30 and Rh1 or 15%FBS+DME for SJ-G2. After seven days exposure to drug containing media, the nuclei were released by treating the cells with a hypotonic solution followed by a detergent. The nuclei were then counted with a Coulter Counter. The results of the experiments were graphed and the IC₅₀ (drug concentration producing 50% inhibition of growth) for each drug was determined by extrapolation. Because the IC50s varied slightly from experiment to experiment, two values that bracketed the IC50 of each drug were used in the interaction studies. The point of maximum interaction between two drugs occurs when they are present in a 1:1 ratio if the isobole is of standard shape. Therefore, each of the three approximate IC₅₀ concentrations of CCI-779 was mixed in a 1:1 ratio with each of three approximated IC₅₀s of gemcitabine or 5-FU. This resulted in nine 1:1 combinations of drugs in each experiment plus three IC₅₀ concentrations for CCI-779 and the other drug. This protocol usually resulted in at least one combination for each drug containing an IC₅₀ value. The 1:1 combination of IC₅₀ concentrations for CCI-779 and each chemotherapy drug was then used to calculate additivity, synergism, or antagonism using Berenbaum's formula: x/X₅₀+y/Y₅₀, =1,<1,>1. If the three concentrations of CCl-779 tested alone didn't produce an IC that matched any of the three ICs of the other compound tested alone, all the 1:1 combinations were checked to see if their ICs fell between the appropriate ICs of drugs tested singly. If they did, the effect was considered additive.

The results obtained in the *in vitro* standard pharmacological test procedure showed that in no case did the combinations yield less than a 50% inhibition of growth indicating that the combinations were at least additive and produced no evidence of antagonism.

Female CBA/CaJ mice (Jackson Laboratories, Bar Harbor, ME), 4 weeks of age, were immune-deprived by thymectomy, followed 3 weeks later by whole-body irradiation (1200 cGy) using a ¹³⁷Cs source. Mice received 3 x 10⁶ nucleated bone marrow cells within 6-8 h of irradiation. Tumor pieces of approximately 3 mm³ were implanted in the space of the dorsal lateral flanks of the mice to initiate tumor growth. Tumor-bearing mice were randomized into groups of seven prior to initiating therapy. Mice bearing tumors each received drug when tumors were approximately 0.20-1 cm in diameter. Tumor size was determined at 7-day intervals using digital Vernier calipers interfaced with a computer. Tumor volumes were calculated assuming tumors to be spherical using the formula [(π/6) x *d*³], where d is the mean diameter. CCI-779 was given on a schedule of 5 consecutive days for 2 weeks with this cycle repeated every 21 days for 3 cycles. This resulted in CCl-779 being given on days 1-5, 8-12 (cycle 1); 21-25, 28-32 (cycle 2); and 42-46, 49-53 (cycle 3). The schedule of the other chemotherapy drug for each study was as follows:
Gemcitabine on days 1, 4, 8 in cycle 1 only

The combination of CCI-779 and gemcitabine was evaluated in a human colon (GC3) mouse xenograft test procedure. In this test procedure, CCI-779 was given daily x 5 for 2 consecutive weeks every 21 days for 3 cycles and gemcitabine given on days 1, 4, and 8 in the first cycle only. The presence of CCI-779 did not enhance tumor regression seen in the first cycle with gemcitabine treatment. However, groups treated with CCI-779 were delayed in the time required to reach 2-3x the original pretreatment tumor volume (versus gemcitabine alone), indicating that there was at least an additive benefit derived from the combination treatment.

Based on the results of these standard pharmacological test procedures, combinations of an mTOR inhibitor plus an antimetabolite chemotherapeutic agent are useful as antineoplastic therapy. More particularly, these combinations useful in treating treatment of renal carcinoma, soft tissue sarcoma, breast cancer, neuroendocrine tumor of the lung, cervical cancer, uterine cancer, head and neck cancer, glioma, non-small cell lung cancer, prostate cancer, pancreatic cancer, lymphoma, melanoma, small cell lung cancer, ovarian cancer, colon cancer, esophageal cancer, gastric cancer, leukemia, colorectal cancer, and unknown primary cancer. As these combinations contain at least two active antineoplastic agents, the use of such combinations also provides for the use of combinations of each of the agents in which one or both of the agents is used at subtherapeutically effective dosages, thereby lessening toxicity associated with the individual chemotherapeutic agent.

In providing chemotherapy, multiple agents having different modalities of action are typically used as part of a chemotherapy "cocktail." It is anticipated that the combinations of this invention will be used as part of a chemotherapy cocktail that may contain one or more additional antineoplastic agents depending on the nature of the neoplasia to be treated. For example, this invention also covers the use of the mTOR inhibitor/antimetabolite combination used in conjunction with other chemotherapeutic agents, such as alkylating agents (i.e., cisplatin, carboplatin, streptazoin, melphalan, chlorambucil, carmustine, methclorethamine, lomustine, bisulfan, thiotepa, ifofamide, or cyclophosphamide); hormonal agents (i.e., estramustine, tamoxifen, toremifene, anastrozole, or letrozole); antibiotics (i.e., plicamycin, bleomycin, mitoxantrone, idarubicin, dactinomycin, mitomycin, doxorubicin, or daunorubicin); immunomodulators (i.e., interferons, IL-2, or BCG); antimitotic agents (i.e., vinblastine, vincristine, teniposide, or vinorelbine); topoisomerase inhibitors (i.e., topotecan, irinotecan, or etoposide); and other agents (i.e., hydroxyurea, trastuzumab, altretamine, retuximab, paclitaxel, docetaxel, L-asparaginase, or gemtuzumab ozogamicin).

As used in this invention, the combination regimen can be given simultaneously or can be given in a staggered regimen, with the mTOR inhibitor being given at a different time during the course of chemotherapy than the antimetabolite. This time differential may range from several minutes, hours, days, weeks, or longer between administration of the two agents. Therefore, the term combination does not necessarily mean administered at the same time or as a unitary dose, but that each of the components are administered during a desired treatment period. The agents may also be administered by different routes. For example, in the combination of an mTOR inhibitor plus an antimetabolite, it is anticipated that the mTOR inhibitor will be administered orally or parenterally, with parenterally being preferred, while the antimetabolite may be administered parenterally, orally, or by other acceptable means. For the CCI-779 combination with gemcitabine, it is preferred that the gemcitabine be administered parenterally. For the CCI-779 combination with 5-FU and leucovorin, it is preferred that the 5-FU and leucovorin are administered parenterally. These combination can be administered daily, weekly, or even once monthly. As typical for chemotherapeutic regimens, a course of chemotherapy may be repeated several weeks later, and may follow the same timeframe for administration of the two agents, or may be modified based on patient response.

Accordingly this invention also provides a product comprising rapamycin 42-ester with 3-hydroxy-2-(hydroxymethyl)-2-methylpropionic acid [CCI-779] as an mTOR inhibitor and an antimetabolite antineoplastic agent selected from 5-fluorouracil and gemcitabine as a combined preparation for simultaneous, separate or sequential use in the treatment of a neoplasm in a mammal.

As typical with chemotherapy, dosage regimens are closely monitored by the treating physician, based on numerous factors including the severity of the disease, response to the disease, any treatment related toxicities, age, health of the patient, and other concomitant disorders or treatments.

Based on the results obtained with the representative CCI-779 plus antimetabolite combinations, it is projected that the initial i.v. infusion dosage of the mTOR inhibitor will be between 0.1 and 100 mg/m², with between 2.5 and 70 mg/m² being preferred. It is also preferred that the mTOR inhibitor be administered by i.v., typically over a 30 minute period, and administered about once per week. The initial dosages of the antimetabolite component will depend on the component used, and will be based initially on physician experience with the agents chosen.

Based on the results obtained with the CCl-779 plus antimetabolite combinations, it is projected that for the mTOR inhibitor plus gemcitabine combination, the initial i.v. infusion dosage of the mTOR inhibitor will be between 0.1 and 100 mg/m², with between 2.5 and 70 mg/m² being preferred, and the initial i.v. infusion dosage of gemcitabine will be between 400 and 1500 mg/m², with between 800 and 1000 mg/m² being preferred. It is initially projected that patients will receive a 30 minute i.v. infusion of the mTOR inhibitor, followed immediately or preceded by a 30 minute i.v. infusion of gemcitabine on days 1 and 8 of a 21 day treatment cycle. After one or more treatment cycles, the dosages can be adjusted upwards or downwards depending on the results obtained and the side effects observed.

Based on the results obtained, when CCI-779 is used in combination with 5-FU and leucovorin, it is projected that in an mTOR inhibitor plus 5-FU plus leucovorin regimen, the initial i.v. infusion dosage of the mTOR inhibitor will be between 0.1 and 100 mg/m², with between 2.5 and 70 mg/m² being preferred; the initial i.v. infusion dosage of leucovorin will be between 50 and 500 mg/m², with 200 mg/m² being preferred; and the initial i.v. infusion dosage of 5-FU will be between 500 and 7500 mg/m², with between 1000 and 5000 mg/m² being preferred. It is initially projected that the combination will be administered according to the following regimen: patients will receive a 1 hour i.v. infusion of leucovorin once weekly during each 6 week treatment cycle; immediately following each dose of leucovorin, 5-FU is administered as a 24-hour continuous i.v. infusion. The mTOR inhibitor will be administered beginning on day 8, of cycle 1, and will be given once weekly as a 30 minute i.v. infusion. Each 6 week treatment cycle is followed by a 1 week rest before beginning the next 6 week treatment cycle. After one or more treatment cycles, the dosages can be adjusted upwards or downwards depending on the results obtained and the side effects observed.

For commercially available antimetabolites, the existing dosage form can be used, with the dosages divided as need be. Alternatively, such agents or antimetabolites that are not commercially available can be formulated according to standard pharmaceutical practice. Oral formulations containing the active compounds of this invention may comprise any conventionally used oral forms, including tablets, capsules, buccal forms, troches, lozenges and oral liquids, suspensions or solutions. Capsules may contain mixtures of the active compound(s) with inert fillers and/or diluents such as the pharmaceutically acceptable starches (e.g. com, potato or tapioca starch), sugars, artificial sweetening agents, powdered celluloses, such as crystalline and microcrystalline celluloses, flours, gelatins, gums, etc. Useful tablet formulations may be made by conventional compression, wet granulation or dry granulation methods and utilize pharmaceutically acceptable diluents, binding agents, lubricants, disintegrants, surface modifying agents (including surfactants), suspending or stabilizing agents, including, but not limited to, magnesium stearate, stearic acid, talc, sodium lauryl sulfate, microcrystalline cellulose, carboxymethylcellulose calcium, polyvinylpyrrolidone, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, dry starches and powdered sugar. Preferred surface modifying agents include nonionic and anionic surface modifying agents. Representative examples of surface modifying agents include, but are not limited to, poloxamer 188, benzalkonium chloride, calcium stearate, cetosteart alcohol, cetomacrogol emulsifying wax, sorbitan esters, colloidol silicon dioxide, phosphates, sodium dodecylsulfate, magnesium aluminum silicate, and triethanolamine. Oral formulations herein may utilize standard delay or time release formulations to alter the absorption of the active compound(s). The oral formulation may also consist of administering the active ingredient in water or a fruit juice, containing appropriate solubilizers or emulsifiers as needed.

In some cases it may be desirable to administer the compounds directly to the airways in the form of an aerosol.

The compounds may also be administered parenterally or intraperitoneally. Solutions or suspensions of these active compounds as a free base or pharmacologically acceptable salt can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparation contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils.

For the purposes of this disclosure, transdermal administrations are understood to include all administrations across the surface of the body and the inner linings of bodily passages including epithelial and mucosal tissues. Such administrations may be carried out using the present compounds, or pharmaceutically acceptable salts thereof, in lotions, creams, foams, patches, suspensions, solutions, and suppositories (rectal and vaginal).

Transdermal administration may be accomplished through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semi-permeable membrane covering a reservoir containing the active ingredient with or without a carrier, or a matrix containing the active ingredient. Other occlusive devices are known in the literature.

Suppository formulations may be made from traditional materials, including cocoa butter, with or without the addition of waxes to alter the suppository's melting point, and glycerin. Water soluble suppository bases, such as polyethylene glycols of various molecular weights, may also be used.

## Claims

1. Use of a combination consisting of rapamycin 42-ester with 3-hydroxy-2-(hydroxymethyl)-2-mothylpropionic acid [CCI-779]; a compound selected from 5-fluorouracil and gemcitabine; and optionally a biochemical modifying agent selected from leucovorin and levofolinate in the preparation of a medicament for treating a neoplasm in a mammal.

2. Use according to claim 1 wherein the neoplasm is treated with subtherapeutically effective dosages of either CCI-779, the compound selected from 5-fluorouracil and gemcitabine, or both.

3. Use according to claim 2 in which the neoplasm is treated with CCI-779 in subtherapeutically effective dosages.

4. Use according to claim 2 or claim 3 In which the neoplasm is treated with the compound selected from 5-fluorouracil and gemcitabine in subtherapeutically effective dosages.

5. Use according to any one of claims 1 to 4, wherein the neoplasm is one of the following:
renal cancer; soft tissue sarcoma; breast cancer; a neuroendocrine tumor of the lung; cervical cancer, uterine cancer: a head and neck cancer, glioma; non-small cell lung cancer, prostate cancer; pancreatic cancer; lymphoma; melanoma; small cell lung cancer, ovarian cancer, colon cancer; esophageal cancer; gastric cancer; leukemia; colorectal cancer; or unknown primary cancer.

6. Use according to any one of claims 1 to 5, wherein the combination includes a biochemical modifying agent selected from leucovorin and levofolinate.

7. Use according to any one of claims 1 to 5 in which the combination includes gemcitabine.

8. Use according to claim 7. wherein the neoplasm is pancreatic cancer.

9. Use according to any one of claims 1 to 5 in which the combination includes 5-fluorouracil.

10. Use according to claim 9, in which the combination includes a biochemical modifying agent selected from leucovorin and levofolinate.

11. Use according to any one of claims 1 to 5 wherein the medicament comprises an effective amount of a combination of rapamycin 42-ester with 3-hydroxy-2-(hydroxymethyl)-2-methylpropionic acid, 5-fluorouracil, and leucovorin.

12. Use according to any one of claims 9 to 11, wherein the neoplasm is colorectal cancer.

13. An antineoplastic combination which comprises an effective amount of rapamycin 42-ester with 3-hydroxy-2-(hydroxymethyl)-2-methylpropionic acid; a compound selected from 5-fluorouracil and gemcitabine; and optionally a biochemical modifying agent selected from leucovorin and levofolinate.

14. The combination of claim 13, which includes a biochemical modifying agent selected from leucovorin and levofolinate.

15. A product comprising rapamycin 42-ester with 3-hydroxy-2-(hydroxymethyl)-2-methylpropionic acid; a compound selected from 5-fluorouracil and gemcitabine; and optionally a biochemical modifying agent selected from leucovorin and levofolinate as a combined preparation for simultaneous, separate or sequential use in the treatment of a neoplasm in a mammal.

16. A product as claimed in claim 15 which includes a biochemical modifying agent selected from leucovorin and levofolinate.

17. A product as claimed in claim 15 or claim 16 wherein the neoplasm is one of the following:
renal cancer; soft tissue sarcoma; breast cancer; a neuroendocrine tumor of the lung; cervical cancer; uterine cancer; a head and neck cancer; glioma; non-small cell lung cancer; prostate cancer; pancreatic cancer; lymphoma; melanoma; small cell lung cancer; ovarian cancer; colon cancer; esophageal cancer; gastric cancer; leukemia; colorectal cancer; or unknown primary cancer.

18. Use of rapamycin 42-ester with 3-hydroxy-2-(hydroxymethyl)-2-methylpropionic acid in the preparation of a medicament for treating a neoplasm in a mammal for simultaneous, separate or sequential use with a compound selected from 5-fluorouracil and gemcitabine; and optionally a biochemical modifying agent selected from leucovorin and levofolinate in the treatment of a neoplasm in a mammal.

19. Use of compound selected from 5-fluorouracil and gemcitabine in the preparation of a medicament for treating a neoplasm in a mammal for simultaneous, separate or sequential use with rapamycin 42-ester with 3-hydroxy-2-(hydroxymethyl)-2-methylpropionic acid and optionally a biochemical modifying agent selected from leucovorin and levofolinate in the treatment of a neoplasm in a mammal.

## Patentansprüche

1. Verwendung einer Kombination, bestehend aus Rapamycin 42-Ester mit 3-Hydroxy-2-(Hydroxymethyl)-2-Methylpropionsäure [CCI-779], einer aus 5-Fluorouracil und Gemcitabin ausgewählten Verbindung, optional einem aus Leukovorin und Levofolinat ausgewählten, biochemischen, modifizierenden Agens, bei der Zubereitung eines Medikaments zur Behandlung eines Neoplasmas bei einem Säugetier.

2. Verwendung nach Anspruch 1, wobei das Neoplasma mit subtherapeutisch wirksamen Dosierungen entweder von CCI-779 oder der aus 5-Fluorouracil und Gemcitabin ausgewählten Verbindung oder von beiden behandelt wird.

3. Verwendung nach Anspruch 2, wobei das Neoplasma mit CCI-779 in subtherapeutisch wirksamen Dosierungen behandelt wird.

4. Verwendung nach Anspruch 2 oder Anspruch 3, wobei das Neoplasma mit der aus 5-Fluorouracil und Gemcitabin ausgewählten Verbindung in subtherapeutisch wirksamen Dosierungen behandelt wird.

5. Verwendung nach irgendeinem der Ansprüche 1 bis 4, wobei das Neoplasma eines der Folgenden ist: Nierenkarzinom, Weichteilsarkom, Brustkrebs, ein neuroendokriner Tumor der Lunge, Zervixkarzinom, Uteruskarzinom, Kopf- und Nackenkarzinom, Gliom, nichtkleinzelliges Lungenkarzinom, Prostatakarzinom, Pankreaskarzinom, Lymphom, Melanom, kleinzelliges Lungenkarzinom, Ovarialkarzinom, Kolonkarzinom, Ösophaguskarzinom, Magenkarzinom, Leukämie, kolorektales oder unbekanntes Primärkarzinom.

6. Verwendung nach irgendeinem der Ansprüche 1 bis 5, wobei die Kombination ein biochemisches modifizierendes Agens umfasst, das aus Leukovorin und Levofolinat ausgewählt ist.

7. Verwendung nach irgendeinem der Ansprüche 1 bis 5, wobei die Kombination Gemcitabin einschließt.

8. Verwendung nach Anspruch 7, wobei das Neoplasma ein Pankreaskarzinom ist.

9. Verwendung nach irgendeinem der Ansprüche 1 bis 5, wobei die Kombination 5-Fluorouracil einschließt.

10. Verwendung nach Anspruch 9, wobei die Kombination ein biochemisches modifizierendes Agens einschließt, das aus Leukovorin und Levofolinat ausgewählt ist.

11. Verwendung nach irgendeinem der Ansprüche 1 bis 5, wobei das Medikament eine wirksame Menge einer Kombination aus Rapamycin 42-Ester mit 3-Hydroxy-2-(Hydroxymethyl)-2-Methylpropionsäure, 5-Fluorouracil und Leukovorin umfasst.

12. Verwendung nach irgendeinem der Ansprüche 9 bis 11, wobei das Neoplasma ein kolorektales Karzinom ist.

13. Antineoplastische Kombination, die eine wirksame Menge von Rapamycin 42-Ester mit 3-Hydroxy-2-(Hydroxymethyl)-2-Methylpropionsäure, einer aus 5-Fluorouracil und Gemcitabin ausgewählten Verbindung und optional eines aus Leukovorin und Levofolinat ausgewählten biochemischen modifizierenden Agens umfasst.

14. Die Kombination nach Anspruch 13, die ein aus Leukovorin und Levofolinat ausgewähltes biochemisches modifizierendes Agens umfasst.

15. Ein Produkt, das Rapamycin 42-Ester mit 3-Hydroxy-2-(Hydroxymethyl)-2-Methylpropionsäure, eine aus 5-Fluorouracil und Gemcitabin ausgewählte Verbindung und optional ein aus Leukovorin und Levofolinat ausgewähltes biochemisches modifizierendes Agens umfasst als kombinierte Zubereitung für die gleichzeitige, getrennte oder aufeinander folgende Verwendung zur Behandlung eines Neoplasmas bei einem Säugetier.

16. Produkt nach Anspruch 15, das ein aus Leukovorin und Levofolinat ausgewähltes biochemisches modifizierendes Agens einschließt.

17. Produkt nach Anspruch 15 oder Anspruch 16, wobei das Neoplasma eines der Folgenden ist: Nierenkarzinom, Weichteilsarkom, Brustkrebs, ein neuroendokriner Tumor der Lunge, Zervixkarzinom, Uteruskarzinom, Kopf- und Nackenkarzinom, Gliom, nichtkleinzelliges Lungenkarzinom, Prostatakarzinom, Pankreaskarzinom, Lymphom, Melanom, kleinzelliges Lungenkarzinom, Ovarialkarzinom, Kolonkarzinom, Ösophaguskarzinom, Magenkarzinom, Leukämie, kolorektales oder unbekanntes Primärkarzinom.

18. Verwendung von Rapamycin 42-Ester mit 3-Hydroxy-2-(Hydroxymethyl)-2-Methylpropionsäure bei der Zubereitung eines Medikaments zur Behandlung eines Neoplasmas bei einem Säugetier zur gleichzeitigen, getrennten oder aufeinander folgenden Verwendung mit einer aus 5-Fluorouracil und Gemcitabin ausgewählten Verbindung und optional eines aus Leukovorin und Levofolinat ausgewählten biochemischen modifizierenden Agens zur Behandlung eines Neoplasmas eines Säugetiers.

19. Verwendung einer aus 5-Fluorouracil und Gemcitabin ausgewählten Verbindung bei der Zubereitung eines Medikaments zur Behandlung eines Neoplasmas bei einem Säugetier zur gleichzeitigen, getrennten oder aufeinander folgenden Verwendung mit Rapamycin 42-Ester mit 3-Hydroxy-2-(Hydroxymethyl)-2-Methylpropionsäure und optional eines aus Leukovorin und Levofolinat ausgewählten biochemischen modifizierenden Agens zur Behandlung eines Neoplasmas eines Säugetiers.

## Revendications

1. Utilisation d'une combinaison constituée d'ester de la rapamycine 42 avec l'acide 3-hydroxy-2-(hydroxyméthyl)-2-méthylpropionique [CCI-779] ; un composé choisi parmi le 5-fluorouracil et la gemcitabine ; et éventuellement un agent biochimique de modification choisi parmi la leucovorine et le lévofolinate dans la préparation d'un médicament pour traiter un néoplasme chez un mammifère.

2. Utilisation selon la revendication 1, dans laquelle le néoplasme est traité avec des doses sous-thérapeutiquement efficaces soit de CCI-779, le composé choisi parmi le 5-fluorouracil et la gemcitabine, ou les deux.

3. Utilisation selon la revendication 2, dans laquelle le néoplasme est traité avec du CCI-779 en doses sous-thérapeutiquement efficaces.

4. Utilisation selon la revendication 2 ou la revendication 3, dans laquelle le néoplasme est traité avec le composé choisi parmi le 5-fluorouracil et la gemcitabine en doses sous-thérapeutiquement efficaces.

5. Utilisation selon l'une quelconque des revendications 1 à 4, dans laquelle le néoplasme est l'un parmi les suivants :
cancer du rein ; sarcome des tissus mous ; cancer du sein ; une tumeur neuroendocrine du poumon ; cancer du col ; cancer de l'utérus ; cancer de la tête et du cou ; gliome ; cancer du poumon non à petites cellules ; cancer de la prostate ; cancer du pancréas ; lymphome ; mélanome ; cancer du poumon à petites cellules ; cancer de l'ovaire ; cancer du côlon ; cancer de l'oesophage; cancer gastrique ; leucémie ; cancer côlorectal ; ou cancer primaire inconnu.

6. Utilisation selon l'une quelconque des revendications 1 à 5, dans laquelle la combinaison inclut un agent biochimique de modification choisi parmi la leucovorine et le lévofolinate.

7. Utilisation selon l'une quelconque des revendications 1 à 5, dans laquelle la combinaison inclut la gemcitabine.

8. Utilisation selon la revendication 7, dans laquelle le néoplasme est le cancer du pancréas.

9. Utilisation selon l'une quelconque des revendications 1 à 5, dans laquelle la combinaison inclut le 5-fluorouracil.

10. Utilisation selon la revendication 9, dans laquelle la combinaison inclut un agent biochimique de modification choisi parmi la leucovorine et le lévofolinate.

11. Utilisation selon l'une quelconque des revendications 1 à 5, dans laquelle le médicament comprend une quantité efficace d'une combinaison d'ester de la rapamycine 42 avec l'acide 3-hydroxy-2-(hydroxyméthyl)-2-méthylpropionique, le 5-fluorouracil, et la leucovorine.

12. Utilisation selon l'une quelconque des revendications 9 à 11, dans laquelle le néoplasme est le cancer côlorectal.

13. Combinaison antinéoplasique qui comprend une quantité efficace d'ester de la rapamycine 42 avec l'acide 3-hydroxy-2-(hydroxyméthyl)-2-méthylpropionique ; un composé choisi parmi le 5-fluorouracil et la gemcitabine ; et éventuellement un agent biochimique de modification choisi parmi la leucovorine et le lévofolinate.

14. Combinaison selon la revendication 13, qui inclut un agent biochimique de modification choisi parmi la leucovorine et le lévofolinate.

15. Produit comprenant l'ester de la rapamycine 42 avec l'acide 3-hydroxy-2-(hydroxyméthyl)-2-méthylpropionique ; un composé choisi parmi le 5-fluorouracil et la gemcitabine ; et éventuellement un agent biochimique de modification choisi parmi la leucovorine et le lévofolinate sous la forme d'une préparation combinée pour l'utilisation simultanée, séparée ou séquentielle dans le traitement d'un néoplasme chez un mammifère.

16. Produit tel que revendiqué dans la revendication 15, qui inclut un agent biochimique de modification choisi parmi la leucovorine et le lévofolinate.

17. Produit tel que revendiqué selon la revendication 15 ou la revendication 16, dans lequel le néoplasme est l'un des suivants :
cancer du rein ; sarcome des tissus mous ; cancer du sein ; une tumeur neuroendocrine du poumon ; cancer du col ; cancer de l'utérus ; cancer de la tête et du cou ; gliome ; cancer du poumon non à petites cellules ; cancer de la prostate ; cancer du pancréas ; lymphome ; mélanome ; cancer du poumon à petites cellules ; cancer de l'ovaire ; cancer du côlon ; cancer de l'oesophage; cancer gastrique ; leucémie ; cancer côlorectal ; ou cancer primaire inconnu.

18. Utilisation d'ester de la rapamycine 42 avec de l'acide 3-hydroxy-2-(hydroxyméthyl)-2-méthylpropionique dans la préparation d'un médicament pour traiter un néoplasme chez un mammifère pour l'utilisation simultanée, séparée ou séquentielle avec un composé choisi parmi le 5-fluorouracil et la gemcitabine ; et éventuellement un agent biochimique de modification choisi parmi la leucovorine et le lévofolinate dans le traitement d'un néoplasme chez un mammifère.

19. Utilisation d'un composé choisi parmi le 5-fluorouracil et la gemcitabine dans la préparation d'un médicament pour le traitement d'un néoplasme chez un mammifère pour l'utilisation simultanée, séparée ou séquentielle avec l'ester de la rapamycine 42 avec l'acide 3-hydroxy-2-(hydroxyméthyl)-2-méthylpropionique et éventuellement un agent biochimique de modification choisi parmi la leucovorine et le lévofolinate dans le traitement d'un néoplasme chez un mammifère.
